# EUROPÄISCHE PATENTSCHRIFT

(11) **EP 3 470 579 B1**
(45) Veröffentlichungstag und Bekanntmachung des Hinweises auf die Patenterteilung: **26.06.2024**
(21) Anmeldenummer: 18197326.4
(22) Anmeldetag: 27.09.2018
(51) Int. Cl.: E02D 5/18, E02D 33/00, G01K 1/02, G01N 33/38

(54) **VERFAHREN ZUM AUFFINDEN VON FEHLSTELLEN IN SCHLITZWÄNDEN ODER ZUM ÜBERPRÜFEN DER DICHTIGKEIT EINER SCHLITZWANDFUGE UND THERMISCHES SCHLITZWANDFUGENKONTROLLSYSTEM**
METHOD FOR DETERMINING DEFECTIVE POINTS IN SLOT WALLS OR FOR TESTING THE FLUID TIGHTNESS OF A SLOT WALL JOINT AND THERMAL SLOT WALL JOINT TEMPERATURE CONTROL SYSTEM
PROCÉDÉ DE REPÉRAGE DES DÉFAUTS DANS LES PAROIS CONTINUES OU DE VÉRIFICATION DE L'ÉTANCHÉITÉ D'UN JOINT DE LA PAROI CONTINUE ET SYSTÈME DE CONTRÔLE THERMIQUE DES JOINTS DE LA PAROI CONTINUE

(30) Priorität: 10.10.2017 DE 102017123528
(43) Veröffentlichungstag der Anmeldung: 17.04.2019
(73) Patentinhaber: PORR Spezialtiefbau GmbH, 80807 München (DE)
(72) Erfinder: Kindler, Arne, 14621 Schönwalde-Glien (DE)
(74) Vertreter: Eder Schieschke & Partner mbB

(56) Entgegenhaltungen:
- WO-A1-2015/145263
- WO-A2-2008/007025
- DE-A1- 19 921 256
- DE-A1-102013 205 765
- DE-U1- 9 001 679
- FR-A1- 2 836 548
- FR-A1- 2 953 943
- GB-A- 2 182 452
- US-A- 4 943 930
- US-A- 5 399 854
- US-A1- 2011 200 068

## Beschreibung

Die vorliegende Erfindung betrifft ein Verfahren zum Auffinden von Fehlstellen gemäß Anspruch 1 sowie ein Verfahren zum Überprüfen der Dichtigkeit einer Schlitzwandfuge gegenüber dem Eindringen von Flüssigkeiten in eine Baugrube gemäß Anspruch 10. Die vorliegende Erfindung betrifft auch ein thermisches Schlitzwandfugenkontrollsystem gemäß Anspruch 12.

Schlitzwände werden im Bau gerne zur Absicherung einer Baugrube eingesetzt. Hierbei ist es wichtig, dass die Schlitzwände ausreichend gegen das Eindringen von Flüssigkeiten, wie Grundwasser, abgedichtet sind. Schlitzwände werden in der Regel hergestellt, indem ein vertikaler Schlitz bis zu einer gewünschten Tiefe mit einem Schlitzwandgreifer aus dem Boden ausgehoben wird. Dieser Schlitz im Boden wird anschließend mit Beton zu einer sog. Schlitzwandlamelle ausgegossen. Neben einer solchen Schlitzwandlamelle werden in vertikaler Richtung parallele weitere Schlitze in den Boden eingebracht, die ebenso mit Beton ausgegossen werden. Um den Bereich zwischen den Schlitzwandlamellen, die sog. Schlitzwandfugen, gegen Flüssigkeit, wie Grundwasser, abzudichten, wird zwischen jeweils zwei Schlitzwandlamellen ein sog. Schlitzwandfugenband eingebracht, das in seinen Randbereichen jeweils in beide Schlitzwandlamellen mit einbetoniert wird. Damit nur der Randbereich des Fugenbandes von dem Beton einer Schlitzwandlamelle umschlossen wird, wird ein sogenanntes Abschalelement zum Halten des Fugenbandes eingesetzt. Es kann hier bei der Betonabbindung im Bereich der Schlitzwandfugenbänder zu unerwünschten Fehlstellen kommen, durch die Flüssigkeit bzw. Grundwasser in die abzusichernde Baugrube eindringen kann.

Deshalb ist die Prüfung der Schlitzwandfugen (technische Wasserundurchlässigkeit) vor der eigentlichen Grundwasserabsenkung (Pumpversuch) in einer Baugrube eine wesentliche Qualitätssicherungsmaßnahme. In diesem Zusammenhang offenbart die DE 10 2013 205 765 A1 ein Qualitätssicherungssystem für Schlitzwandfugen. Für die Nutzung dieses Qualitätssicherungssystems ist es erforderlich, in den laufenden Bauablauf einzugreifen. Damit sind Unterbrechungen und Störungen des Bauablaufs sowie Zusatzkosten verbunden.

Die US 2011/200068 A1 betrifft ein System zum Überwachen der Formgebung eines festen Objekts mit einem Sensorstrang, der in einer Formungsstruktur vor dem Aushärtungsprozess positionierbar ist, und einer Kommunikationsleitung, die sich entlang einer Strangachse zwischen einem ersten und einem zweiten Ende erstreckt. Die Kette umfasst ferner mehrere Sensoren, die mit der Kommunikationsleitung zwischen den Enden verbunden sind, und jeder Sensor wird an einer festgelegten Position auf der Leitung montiert. Hier wird keine faseroptischer Temperaturmessaufnehmer innerhalb eines Hohlraums eines Fugenbands verwendet.

Die DE 90 01 679 U1 betrifft eine Vorrichtung zum Lösen von Abschalelementen zur Schlitzwandherstellung vom erhärteten Beton, dadurch gekennzeichnet, dass an der dem Beton zugewandten Fläche des Abschalelementes mindestens eine Hubvorrichtung angeordnet ist, die bei Betätigung zwischen dem Abschalelement und dem erhärteten Beton Kräfte erzeugt, so dass das Abschalelement vom erhärteten Beton abgedrückt wird, dass die Hubvorrichtung aus mindestens einem hydraulisch oder pneumatisch betätigbaren Kolben besteht, der in einer Aussparung des Abschalelementes angeordnet und geführt ist, und dass die dem Beton zugewandte Fläche des Kolbens in seiner Ausgangsposition im Wesentlichen in einer Ebene mit der Schalfläche des Abschalelementes liegt und die Aussparung abdichtet.

Die DE 199 21 256 A1 betrifft ein Verfahren und eine Vorrichtung zum Monitoring von Temperaturverteilungen und/oder Temperaturanomalien auf der Basis verteilter faseroptischer Temperatursensorik sowie neuartige Anwendungen derartiger Verfahren.

Es wäre deshalb wünschenswert, ein Verfahren zum Auffinden von Fehlstellen in Schlitzwänden bzw. ein Verfahren zum Überprüfen der Dichtigkeit einer Schlitzwandfuge gegenüber dem Eindringen von Flüssigkeiten bereitzustellen, bei dem in den laufenden Bauablauf nicht eingegriffen werden muss, damit Zusatzkosten vermieden werden können.

Dafür werden in der vorliegenden Anmeldung zwei einfache und kostengünstige Verfahren bereitgestellt. Das erste Verfahren (erste Ausführungsform) dient zum einfachen Auffinden von Fehlstellen in Schlitzwänden während der Betonabbindung, wohingegen mit dem zweiten Verfahren (zweite Ausführungsform) die Dichtigkeit einer Schlitzwandfuge gegenüber dem Eindringen von Flüssigkeiten in eine Baugrube nach der Fertigstellung überprüft werden kann.

In der ersten Ausführungsform wird also ein Verfahren zum Auffinden von Fehlstellen in Schlitzwänden, insbesondere im Schlitzwandfugenbereich mindestens einer Schlitzwandlamelle bereitgestellt, das die folgenden Schritte umfasst:
a) Einbringen eines Schlitzwandfugenbandes (im Folgenden nur Fugenband) in einen Schlitz des Bodens in vertikaler Richtung im Randbereich des Schlitzes;
b) Einbringen eines faseroptischen Temperaturmessaufnehmers eines Temperaturmesssystems in den Schlitz des Bodens im Bereich des Fugenbandes;
c) Einbringen von Beton in den Schlitz des Bodens; und
d) Messen des Temperaturverlaufs während des Vorgangs der Betonabbindung,
dadurch gekennzeichnet, dass das Fugenband entlang seiner Erstreckungsrichtung einen Hohlraum aufweist, und dass in Schritt (b) der faseroptische Temperaturmessaufnehmer in den Hohlraum des Fugenbandes eingebracht wird, so dass der faseroptische Temperaturmessaufnehmer im Hohlraum des Fugenbandes verläuft.

Gemäß einer physikalischen Gesetzmäßigkeit ist der tiefenabhängige Temperaturverlauf bei der Betonabbindung dann ein linearer Verlauf, wenn über die gesamte Tiefe der Schlitzwandlamelle der eingebrachte Beton eine konstante Dicke aufweist und auch das Fugenband in jeder Tiefe einschließt. Grund hierfür ist die Hydratationswärmeentwicklung während des Abbindeprozesses des Betons, die proportional zu der Menge bzw. Dicke des eingebrachten Betons ist.

Deshalb wird vorzugsweise der Temperaturverlauf in Schritt d) mit dem theoretisch linearen Temperaturverlauf bei der Betonabbindung verglichen. Dabei wird bei Abweichen des tatsächlich gemessenen Temperaturverlaufs (tiefenabhängig gemessen) von dem theoretisch linearen Temperaturverlauf bei der Betonabbindung auf eine Fehlstelle im Beton geschlossen.

In dem erfindungsgemäßen Verfahren der ersten Ausführungsform wird das Fugenband mithilfe eines sogenannten Abschalelementes in dem Randbereich des Schlitzes gehalten. Das Abschalelement dient zum einen zum Halten des Fugenbandes, zum anderen verhindert es, dass das gesamte Fugenband mit einbetoniert wird. Auf diese Weise kann nur ein Teil des Fugenbandes entlang seiner Erstreckungsrichtung von dem Beton umschlossen werden, und es steht ein weiterer Teil zum Einbetonieren in eine benachbarte Schlitzwandlamelle zur Verfügung. Das Fugenband verläuft in seiner Erstreckungsrichtung vorzugsweise parallel zu der Erstreckungsrichtung des Schlitzes, insbesondere über die gesamte Tiefe des Schlitzes. Dadurch wird gewährleistet, dass in der gesamten Tiefe einer Schlitzwandlamelle ein Fugenband mit einbetoniert wird, das die Schlitzwandfuge zwischen zwei Schlitzwandlamellen schließen und damit abdichten kann.

In einer weiteren Ausgestaltung des Verfahrens der ersten Ausführungsform ist es bevorzugt, dass der Temperaturmessaufnehmer sich parallel zu dem Fugenband im Wesentlichen über die gesamte Länge des Fugenbandes erstreckt. Durch die Erstreckung des Temperaturmessaufnehmers über die gesamte Länge des Fugenbandes ist sichergestellt, dass eine Fehlstelle über den gesamten Bereich bzw. die gesamte Tiefe der Schlitzwandfuge aufgefunden werden kann.

Dabei erfolgt das Messen des Temperaturverlaufs sowohl über den Zeitraum der Betonabbindung als auch tiefenabhängig. Findet die Betonabbindung in jeder Tiefe auf die gleiche Weise unter Abgabe von gleichmäßig viel Hydratationswärme statt, so erhält man in einem Diagramm bei Auftragen der Temperatur gegen die Tiefe der Schlitzwandlamelle eine Gerade. Misst man in verschiedenen Zeitintervallen während der Betonabbindung die Temperatur tiefenabhängig, so können mögliche Fehlstellen dadurch aufgefunden werden, dass an den Stellen, an denen nicht ausreichend Beton das Fugenband umschließt, weniger Hydratationswärme abgeben wird. Auf diese Weise kann auf eine Fehlstelle geschlossen werden.

Das Fugenband weist einen entlang seiner Erstreckungsrichtung verlaufenden Hohlraum auf. Dies hat den Vorteil, dass in diesen Hohlraum der Temperaturmessaufnehmer eingebracht werden kann, sodass er im Hohlraum des Fugenbandes in vertikaler Richtung verläuft. Dadurch ist der Temperaturmessaufnehmer im Idealfall über die gesamte Tiefe gleich weit von dem Beton entfernt.

Das Fugenband ist vorzugsweise aus Kunststoff, insbesondere einem thermoplastischen Kunststoff, wie z.B. PVC-P.

Ist eine Schlitzwandlamelle mit einem Schlitzwandfugenband bereits in den Boden eingebracht, so kann in einen weiteren Schlitz im Boden, der benachbart und parallel dazu verlaufend im Boden ist, ebenso Beton eingebracht werden. Unter "parallel verlaufend" soll hierin verstanden werden, dass die vertikale Erstreckungsrichtung der zweiten Schlitzwandlamelle die gleiche wie die der benachbarten ersten Schlitzwandlamelle ist. Auf die gleiche Weise kann auch eine dritte, vierte, fünfte, usw. Schlitzwandlamelle hergestellt werden, die jeweils zueinander benachbart und in vertikaler Richtung parallel verlaufend im Boden sind. In vertikaler Richtung verlaufen alle Schlitzwandlamellen einer Schlitzwand vorzugsweise parallel zueinander. Betrachtet man eine Ebene, die senkrecht zur Erstreckungsrichtung der Schlitzwandlamellen aufgespannt ist, dann können drei oder mehr benachbarte Schlitzwände jeweils in einer Linie zueinander angeordnet sein. Sie können aber auch in einem beliebigen Winkel zueinander angeordnet sein.

Wird neben einer Schlitzwandlamelle ein weiterer Schlitz mit Beton zu einer benachbarten Schlitzwandlamelle gefüllt, so ist es bevorzugt, dass dabei ein weiterer Teil des Fugenbandes entlang seiner Erstreckungsrichtung von dem in den weiteren Schlitz eingebrachten Beton umschlossen wird. Somit ist gewährleistet, dass ein Fugenband auf beiden Seiten jeweils von dem Beton der benachbarten Schlitzwandlamellen umschlossen ist und somit die Schlitzwandfuge zwischen den beiden Schlitzwandlamellen abdichtet.

Da bei dem Verfahren der ersten Ausführungsform die Temperaturmessung unabhängig von der Herstellung der Schlitzwandelemente erfolgt, greift es nicht in den Bauablauf ein. Somit kommt es zu keinen planmäßigen Unterbrechungen und Störungen des Bauablaufs. Kommt es zu punktuellen Auffälligkeiten im Temperaturverlauf der Hydratationswärmeentwicklung, kann der Bereich der Auffälligkeit im Vorfeld durch geeignete Maßnahmen saniert werden.

Ebenso wie bei der Betonabbindung des Betons der ersten Schlitzwandlamelle kann auch der Temperaturverlauf tiefen- und zeitabhängig bei der Betonabbindung zur Bildung der zweiten Schlitzwandlamelle gemessen werden. Hier kann auf die gleiche Weise - wie oben beschrieben - auf eine mögliche Fehlstelle geschlossen werden.

Um tatsächlich verifizieren zu können, dass eine Störung des linearen Temperaturverlaufs über die Zeit und über die Tiefe der Schlitzwandlamelle vorliegt, die zu einem Eindringen von Wasser durch die Schlitzwand führen könnte, stellt die vorliegende Erfindung in der zweiten Ausführungsform ein Verfahren zum Überprüfen der Dichtigkeit einer Schlitzwandfuge gegenüber dem Eindringen von Flüssigkeiten, vorzugsweise Grundwasser, in eine Baugrube bereit, die durch die Schlitzwand begrenzt ist, wobei dieses Verfahren die folgenden Schritte umfasst:
(i) Einbringen eines faseroptischen Temperaturmessaufnehmers eines Temperaturmesssystems in den Bereich der Schlitzwandfuge zwischen zwei Schlitzwandlamellen im Wesentlichen über die gesamte Tiefe der Schlitzwand, wobei zusätzlich zum Temperaturmessaufnehmer eine Temperaturerhöhungsvorrichtung vorgesehen ist, die sich parallel zu dem Temperaturmessaufnehmer erstreckt;
(ii) Pumpen von Grundwasser aus der von der Schlitzwand begrenzten Baugrube;
(iii) Messen des tiefenabhängigen Temperaturverlaufs mit Hilfe des Temperaturmessaufnehmers nach Schritt (ii),

wobei vor der Messung des tiefenabhängigen Temperaturverlaufs in Schritt (iii) eine Aufheizphase stattfindet,
dadurch gekennzeichnet, dass in Schritt (i) der faseroptische Temperaturmessaufnehmer in einen Hohlraum eines Schlitzwandfugenbandes eingebracht wird.

Unter der "Tiefe der Schlitzwand" wird die vertikale Erstreckungsrichtung verstanden.

In Schritt (i) ist es bevorzugt, den Temperaturmessaufnehmer in den Hohlraum des Fugenbandes über die gesamte Tiefenerstreckung der Schlitzwandfuge einzubringen. Auf diese Weise kann der Temperaturverlauf tiefenabhängig gemessen werden. Auch hier ist bevorzugt, dass das Fugenband eines ist, das den Hohlraum entlang seiner Erstreckungsrichtung aufweist. Der Durchmesser des Hohlraumes herkömmlicher Fugenbänder liegt im Bereich von 1 bis 3 cm. Der Temperaturmessaufnehmer verläuft hierbei im Hohlraum des Fugenbandes.

In einer Ausgestaltung des Verfahrens der zweiten Ausführungsform wird der tiefenabhängige Temperaturverlauf in Schritt (iii) mit dem tiefenabhängigen Temperaturverlauf vor dem Pumpen in Schritt (ii) verglichen. Das heißt in anderen Worten wird vor dem Schritt (ii) der tiefenabhängige Temperaturverlauf gemessen. Dabei erhält man in der Regel eine Gerade, wenn die Temperatur gegen die Tiefe aufgetragen wird. Wird anschließend das Grundwasser aus der von der Schlitzwand begrenzten Baugrube herausgepumpt, so kommt es im Fall einer Fehlstelle im Schlitzwandfugenbereich zu einem Anströmen/Nachströmen des Grundwassers. Aufgrund der damit verbundenen Temperaturänderung im Bereich der Temperaturmesseinrichtung kann festgestellt werden, ob die im Verfahren der ersten Ausführungsform lokalisierte Auffälligkeit im Fugenbandbereich eine Fehlstelle im Sinne der technischen Dichtigkeitsanforderung darstellt.

Strömt in einer Fehlstelle oder undichten Stelle Wasser von außerhalb der Baugrube in die Baugrube, so kommt es in der Regel lokal zu einer Abkühlung, sodass der tiefenabhängige Temperaturverlauf keiner Geraden mehr folgt, sondern in der entsprechenden Tiefe eine Abweichung von der Gerade aufweist.

Die Temperaturerhöhungsvorrichtung erstreckt sich vorzugsweise im Hohlraum des Fugenbandes.

Die Anwesenheit einer Temperaturerhöhungsvorrichtung hat den Vorteil, dass vor der Messung des tiefenabhängigen Temperaturverlaufs in Schritt (iii) gleichzeitig oder nach dem Pumpen eine Aufheizphase stattfinden kann, so dass aufgrund des erhöhten Temperaturgefälles die Abkühlung durch das Anströmen/Nachströmen des Grundwassers in die Baugrube noch leichter detektiert werden kann.

In den beiden Verfahren der ersten und zweiten Ausführungsform handelt es sich um einen faseroptischen Temperaturmessaufnehmer. Unter faseroptischen Temperaturmessaufnehmern werden - Glasfasern verstanden. Dabei handelt es sich um Standartglasfasern, wie sie auch in der Telekomunikation eingesetzt werden. Vorzugweise handelt es sich hierbei um Singlemodefasern mit einem Kerndurchmesser von 9/125 µm bzw. Multimodefaser mit einem Kerndurchmesser 50/125 µm. Dies haben gegenüber klassischen Temperatursensoren den Vorteil, dass keine Umwandlung in ein elektrisches Signal erfolgen muss. Es wird hierbei vorzugsweise die temperaturabhängige Frequenzverschiebung eines durch die Glasfaser geleiteten Laserimpulses detektiert. Durch die gemessene Zeit, die das Laserlicht durch den faseroptischen Temperaturmessaufnehmer zurücklegt kann auf den Ort geschlossen werden. So sind diese in der Lage, gleichzeitig ortsabhängig entlang ihrer Erstreckungsrichtung die Temperatur zu messen.

Die vorliegende Erfindung betrifft auch ein thermisches Schlitzwandfugenkontrollsystem zum Durchführen eines Verfahrens der Ausführungsform 1 oder der Ausführungsform 2, das ein Schlitzwandfugenband mit einem Hohlraum entlang seiner Erstreckungsrichtung und ein faseroptisches Temperaturmesssystem mit einem Temperaturmessaufnehmer im Hohlraum des Schlitzwandfugenbandes umfasst.

Sämtliche bevorzugten Ausführungsformen in den Verfahren der ersten und zweiten Ausführungsform der vorliegenden Erfindung gelten auch für das erfindungsgemäße thermische Schlitzwandfugenkontrollsystem.

### Abbildungen der Figuren:

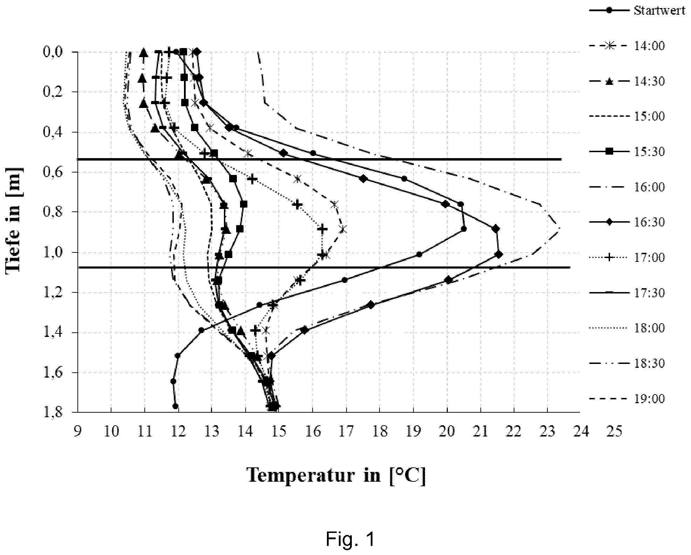

Figur 1 zeigt tiefenabhängige Temperaturkurven, die gemäß dem Verfahren der ersten Ausführungsform zu verschiedenen Zeitpunkten während der Betonabbindung aufgenommen wurden. Dafür wurde aus dem Erdboden ein 1,8 m tiefer Schlitz mit den horizontalen Maßen 300 cm x 80 cm ausgehoben, in den ein 1,8 m langes Schlitzwandfugenband (Typ: DB 3 x 100; Hersteller: Sika) eingebracht wurde. In den in seiner Erstreckungsrichtung verlaufenden Hohlraum des Schlitzwandfugenbandes wurde bis in die Tiefe von 1,8 m ein faseroptischer Temperaturmessaufnehmer eingebracht. Das Schlitzwandfugenband wurde so angeordnet, dass es nur in seinem Randbereich von in den Schlitz eingebrachtem Beton umschlossen wird. Hierfür wurde ein Abschalelement verwendet. Auffällig ist bei der anschließenden Messung der tiefenabhängigen Temperatur, dass es etwa in der Tiefe von 0,9 m zu einer auffälligen Abweichung vom theoretisch linearen Verlauf kommt. Auf diese Weise kann auf eine Fehlstelle in der Schlitzwandfuge geschlossen werden.

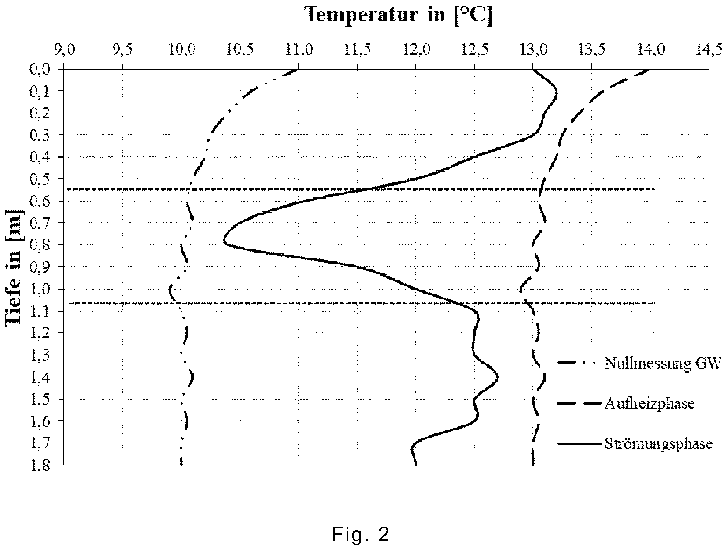

Figur 2 zeigt die tiefenabhängige Temperaturmessung der gleichen Schlitzwandfuge, wie in Figur 1. Die erste Temperaturmessung (Nullmessung GW) erfolgt vor dem Pumpen des Grundwassers (GW) aus der Baugrube, d.h. von der einen Seite der Schlitzwand auf die andere. Anschließend wird die Schlitzwandfuge mit einer Temperaturerhöhungsvorrichtung entlang ihrer Erstreckungsrichtung um etwa 3°C aufgeheizt. Der resultierende Temperaturverlauf ist durch die Kurve rechts (Aufheizphase) wiedergegeben. Auffällig ist, dass bei der Temperaturmessung nach dem Pumpen und dem Aufheizen es etwa in der Tiefe von 0,8 m zu einer viel schnelleren Temperaturabnahme kommt, als im übrigen Bereich (siehe Temperaturkurve in der Mitte (Strömungsphase)). Daraus kann geschlossen werden, dass in dieser Tiefe eine Fehlstelle vorliegt, in der das Grundwasser durch die Schlitzwand dringen kann.

## Patentansprüche

1. Verfahren zum Auffinden von Fehlstellen in Schlitzwänden, insbesondere im Schlitzwandfugenbereich mindestens einer Schlitzwandlamelle, das die folgenden Schritte umfasst:
(a) Einbringen eines Schlitzwandfugenbandes in einen Schlitz des Bodens in vertikaler Richtung im Randbereich des Schlitzes;
(b) Einbringen eines faseroptischen Temperaturmessaufnehmers eines Temperaturmesssystems in den Schlitz des Bodens im Bereich des Schlitzwandfugenbandes;
(c) Einbringen von Beton in den Schlitz des Bodens; und
(d) Messen des Temperaturverlaufs während des Vorgangs der Betonabbindung,
**dadurch gekennzeichnet, dass** das Schlitzwandfugenband entlang seiner Erstreckungsrichtung einen Hohlraum aufweist, und dass in Schritt (b) der faseroptische Temperaturmessaufnehmer in den Hohlraum des Schlitzwandfugenbandes eingebracht wird, so dass der faseroptische Temperaturmessaufnehmer im Hohlraum des Schlitzwandfugenbandes verläuft.

2. Verfahren nach Anspruch 1, wobei der Temperaturverlauf in Schritt (d) mit dem theoretisch linearen Temperaturverlauf bei der Betonabbindung verglichen wird.

3. Verfahren nach Anspruch 2, wobei bei Abweichen des tatsächlich gemessenen Temperaturverlaufs von dem theoretisch linearen Temperaturverlauf bei der Betonabbindung auf eine Fehlstelle im Beton geschlossen wird.

4. Verfahren nach einem der Ansprüche 1 bis 3, wobei ein erster Teil des Schlitzwandfugenbands entlang seiner Erstreckungsrichtung von dem Beton umschlossen wird.

5. Verfahren nach einem der Ansprüche 1 bis 4, wobei der Temperaturmessaufnehmer sich parallel zu dem Schlitzwandfugenband im Wesentlichen über die gesamte Länge des Schlitzwandfugenbandes erstreckt.

6. Verfahren nach Anspruch 5, worin das Messen des Temperaturverlaufs sowohl zeitlich während der Betonabbindung als auch tiefenabhängig erfolgt.

7. Verfahren nach einem der Ansprüche 1 bis 6, wobei in einen weiteren Schlitz im Boden, der benachbart zu dem Schlitz im Boden ist, Beton eingebracht wird.

8. Verfahren nach Anspruch 7, wobei ein zweiter Teil des Schlitzwandfugenbandes entlang seiner Erstreckungsrichtung von dem in den weiteren Schlitz eingebrachten Beton umschlossen wird.

9. Verfahren nach Anspruch 7 oder 8, wobei der Temperaturverlauf bei der Betonabbindung des in den weiteren Schlitz eingebrachten Betons gemessen wird.

10. Verfahren zum Überprüfen der Dichtigkeit einer Schlitzwandfuge gegenüber dem Eindringen von Flüssigkeiten, vorzugsweise Grundwasser, in eine Baugrube, die durch die Schlitzwand begrenzt ist, das die folgenden Schritte umfasst:
(i) Einbringen eines faseroptischen Temperaturmessaufnehmers eines Temperaturmesssystems in den Bereich der Schlitzwandfuge zwischen zwei Schlitzwandlamellen im Wesentlichen über die gesamte Tiefe der Schlitzwand, wobei zusätzlich zum Temperaturmessaufnehmer eine Temperaturerhöhungsvorrichtung vorgesehen ist, die sich parallel zu dem Temperaturmessaufnehmer erstreckt;
(ii) Pumpen von Grundwasser aus der von der Schlitzwand begrenzten Baugrube;
(iii) Messen des tiefenabhängigen Temperaturverlaufs mit Hilfe des Temperaturmessaufnehmers nach Schritt (ii), wobei vor der Messung des tiefenabhängigen Temperaturverlaufs in Schritt (iii) eine Aufheizphase stattfindet,
**dadurch gekennzeichnet, dass** in Schritt (i) der faseroptische Temperaturmessaufnehmer in einen Hohlraum eines Schlitzwandfugenbandes eingebracht wird.

11. Verfahren nach Anspruch 10, wobei der tiefenabhängige Temperaturverlauf in Schritt (iii) mit dem tiefenabhängigen Temperaturverlauf vor dem Pumpen in Schritt (ii) verglichen wird.

12. Thermisches Schlitzwandfugenkontrollsystem zum Durchführen eines Verfahrens nach einem der Ansprüche 1 bis 11, **dadurch gekennzeichnet, dass** es ein Schlitzwandfugenband mit einem Hohlraum entlang seiner Erstreckungsrichtung und ein faseroptisches Temperaturmesssystem mit einem Temperaturmessaufnehmer im Hohlraum des Schlitzwandfugenbandes umfasst.

## Claims

1. Method for finding defects in diaphragm walls, in particular in the diaphragm wall joint region of at least one diaphragm wall panel, comprising the following steps:
(a) introducing a diaphragm wall joint tape into a slot in the ground, in the vertical direction in the edge region of the slot;
(b) introducing a fiber-optic temperature measuring sensor of a temperature measuring system into the slot in the ground, in the region of the diaphragm wall joint tape;
(c) introducing concrete into the slot in the ground, and
(d) measuring the temperature profile during the process of concrete setting,
**characterized in that** the diaphragm wall joint tape has a cavity along its extension direction, and **in that**, and **in that** in step (b), the fiber-optic temperature measuring sensor is introduced into the cavity of the diaphragm wall joint tape, so that the fiber-optic temperature measuring sensor extends in the cavity of the diaphragm wall joint tape.

2. Method according to claim 1, wherein the temperature profile in step (d) is compared with the theoretically linear temperature profile during the concrete setting.

3. Method according to claim 2, wherein it is concluded that there is a defect in the concrete if the actual measured temperature profile deviates from the theoretically linear temperature profile during the concrete setting.

4. Method according to any of claims 1 to 3, wherein a first part of the diaphragm wall joint tape is enclosed along its extension direction by the concrete.

5. Method according to any of claims 1 to 4, wherein the temperature measuring sensor extends in parallel with the diaphragm wall joint tape substantially over the entire length of the diaphragm wall joint tape.

6. Method according to claim 5, wherein the temperature profile is measured both over time during the concrete setting and in a depth-dependent manner.

7. Method according to any of claims 1 to 6, wherein concrete is introduced into a further slot in the ground, which is adjacent to the slot in the ground.

8. Method according to claim 7, wherein a second part of the diaphragm wall joint tape is enclosed along its extension direction by the concrete introduced into the further slot.

9. Method according to either claim 7 or claim 8, wherein the temperature profile is measured during the concrete setting of the concrete introduced into the further slot.

10. Method for checking the tightness of a diaphragm wall joint with respect to the ingress of liquids, preferably groundwater, into an excavation pit which is delimited by the diaphragm wall, comprising the following steps:
(i) introducing a fiber-optic temperature measuring sensor of a temperature measuring system into the region of the diaphragm wall joint between two diaphragm wall panels, substantially over the entire depth of the diaphragm wall, a temperature increasing device being provided in addition to the temperature measuring sensor, which temperature increasing device extends in parallel with the temperature measuring sensor;
(ii) pumping groundwater from the excavation pit delimited by the diaphragm wall;
(iii) measuring the depth-dependent temperature profile with the aid of the temperature measuring sensor after step (ii), a heating phase taking place before the measurement of the depth-dependent temperature profile in step (iii),
**characterized in that** in step (i), the fiber-optic temperature measuring sensor is introduced into a cavity of a diaphragm wall joint tape.

11. Method according to claim 10, wherein the depth-dependent temperature profile in step (iii) is compared with the depth-dependent temperature profile before the pumping in step (ii).

12. Thermal diaphragm wall joint monitoring system for carrying out a method according to any of claims 1 to 11, **characterized in that** it comprises a diaphragm wall joint tape having a cavity along its extension direction, and a fiber-optic temperature measuring system having a temperature measuring sensor in the cavity of the diaphragm wall joint tape.

## Revendications

1. Procédé destiné à repérer des défauts dans des parois moulées, en particulier dans la zone d'un joint de paroi moulée d'au moins une lamelle de paroi moulée, qui comprend les étapes suivantes :
(a) introduction d'un joint d'étanchéité de paroi moulée dans une fente du sol dans la direction verticale dans la zone de bord de la fente ;
(b) introduction d'un capteur de mesure de la température à fibre optique d'un système de mesure de la température dans la fente du sol dans la zone de la bande d'étanchéité de paroi moulée ;
(c) introduction de béton dans la fente du sol ; et
(d) mesure de l'allure de la température pendant le processus de prise du béton,
**caractérisé en ce que** la bande d'étanchéité de paroi moulée présente, le long de sa direction d'extension, un espace creux **et en ce que** dans l'étape (b), le capteur de mesure de la température à fibre optique est introduit dans l'espace creux de la bande d'étanchéité de paroi moulée, en sorte que le capteur de mesure de la température à fibre optique s'étend dans l'espace creux de la bande d'étanchéité de paroi moulée.

2. Procédé selon la revendication 1, dans lequel l'allure de la température dans l'étape (d) est comparée à l'allure de la température linéaire théorique lors de la prise du béton.

3. Procédé selon la revendication 2, dans lequel, lorsque l'allure de la température mesurée effectivement s'écarte de l'allure de la température linéaire théorique lors de la prise du béton, on conclut à un défaut dans le béton.

4. Procédé selon l'une des revendications 1 à 3, dans lequel une première partie de la bande d'étanchéité de paroi moulée est entourée de béton le long de sa direction d'extension.

5. Procédé selon l'une des revendications 1 à 4, dans lequel le capteur de mesure de la température s'étend parallèlement à la bande d'étanchéité de paroi moulée sensiblement sur la toute la longueur de la bande d'étanchéité de paroi moulée.

6. Procédé selon la revendication 5, dans lequel la mesure de l'allure de la température est réalisée à la fois temporellement pendant la prise du béton et en fonction de la profondeur.

7. Procédé selon l'une des revendications 1 à 6, dans lequel du béton est introduit dans une autre fente dans le sol qui est adjacent à la fente dans le sol.

8. Procédé selon la revendication 7, dans lequel une deuxième partie de la bande d'étanchéité de paroi moulée est entourée, le long de sa direction d'extension, du béton introduit dans l'autre fente.

9. Procédé selon la revendication 7 ou 8, dans lequel l'allure de la température est mesurée lors de la prise du béton introduit dans l'autre fente.

10. Procédé destiné à vérifier l'étanchéité d'un joint de paroi moulée par rapport à la pénétration de liquides, de préférence d'eau souterraine, dans une excavation, qui est délimitée par la paroi moulée, qui comprend les étapes suivantes :
(i) introduction d'un capteur de mesure de la température à fibre optique d'un système de mesure de la température dans la zone du joint de paroi moulée entre deux lamelles de paroi moulée, sensiblement sur toute la profondeur de la paroi moulée, un dispositif d'augmentation de la température, qui s'étend parallèlement au capteur de mesure de la température, étant en outre prévu ;
(ii) pompage d'eau souterraine hors de l'excavation délimitée par la paroi moulée ;
(iii) mesure de l'allure de la température en fonction de la profondeur à l'aide du capteur de mesure de la température après l'étape (ii), une phase de chauffage ayant lieu avant la mesure de l'allure de la température en fonction de la profondeur dans l'étape (iii),
**caractérisé en ce que** dans l'étape (i), le capteur de mesure de la température à fibre optique est introduit dans un espace creux d'un joint d'étanchéité de paroi moulée.

11. Procédé selon la revendication 10, dans lequel l'allure de la température en fonction de la profondeur dans l'étape (iii) est comparée à l'allure de la température en fonction de la profondeur avant le pompage dans l'étape (ii).

12. Système thermique de contrôle de joint de paroi moulée, destiné à mettre en oeuvre un procédé selon l'une des revendications 1 à 11,
**caractérisé en ce qu'**il comprend un joint d'étanchéité de paroi moulée comportant un espace creux le long de sa direction d'extension et un système de mesure de la température à fibre optique comportant un capteur de mesure de la température dans l'espace creux de la bande d'étanchéité de paroi moulée.
